# EUROPEAN PATENT SPECIFICATION

(11) **EP 3 918 982 B1**
(45) Date of publication and mention of the grant of the patent: **04.12.2024**
(21) Application number: 21175365.2
(22) Date of filing: 21.05.2021
(51) Int. Cl.: A61B 5/024, A61B 5/0245, A61B 5/00, A61B 5/0205, A61B 5/361

(54) **APPARATUS FOR CLASSIFYING PHOTOPLETHYSMOGRAPHY PULSES AND MONITORING OF CARDIAC ARRHYTHMIAS**
GERAET ZUR KLASSIFIZIERUNG VON PHOTOPLETHYSMOGRAPHISCHEN IMPULSEN UND ÜBERWACHUNG VON HERZARRHYTHMIEN
APPAREIL DE CLASSIFICATION D'IMPULSIONS DE PHOTOPLÉTHYSMOGRAPHIE ET DE SURVEILLANCE D'ARYTHMIES CARDIAQUES

(30) Priority: 02.06.2020 EP 20177900
(43) Date of publication of application: 08.12.2021
(73) Proprietor: CSEM Centre Suisse d'Electronique et de Microtechnique SA - Recherche et Développement, 2002 Neuchâtel (CH)
(72) Inventor: Braun, Fabian, 2000 Neuchâtel (CH); Proença, Martin, 1786 Sugiez (CH); Van Zaen, Jérôme, 1007 Lausanne (CH); Renevey, Philippe, 1005 Lausanne (CH); Lemay, Mathieu, 1350 Orbe (CH)
(74) Representative: P&TS SA (AG, Ltd.)

(56) References cited:
- WO-A1-2007/043903
- US-A1- 2016 345 862
- US-A1- 2018 279 891
- US-A1- 2020 100 693
- SOLOSENKO ANDRIUS ET AL: "Photoplethysmography-Based Method for Automatic Detection of Premature Ventricular Contractions", IEEE TRANSACTIONS ON BIOMEDICAL CIRCUITS AND SYSTEMS, IEEE, US, vol. 9, no. 5, 1 October 2015 (2015-10-01), pages 662 - 669, XP011591052, ISSN: 1932-4545, [retrieved on 20151125], DOI: 10.1109/TBCAS.2015.2477437
- NEHA ET AL: "Photoplethysmography Based Arrhythmia Detection and Classification", 2019 6TH INTERNATIONAL CONFERENCE ON SIGNAL PROCESSING AND INTEGRATED NETWORKS (SPIN), IEEE, 7 March 2019 (2019-03-07), pages 944 - 948, XP033547878, DOI: 10.1109/SPIN.2019.8711737

## Description

### Field

The present disclosure concerns a method for classifying photoplethysmography (PPG) pulses. The present disclosure further concerns a method for detecting atrial fibrillation (AF), classifying arrhythmias and monitoring blood pressure, SpO₂ and sleep and other heart rate variability (HRV) derived parameters. A computer program comprising instructions for implementing the method and an apparatus configured to run the computer program are also disclosed.

### Description of related art

The monitoring of cardiac arrhythmias through PPG is a growing field of research. Interest in AF is high since it is the most common arrhythmia affecting millions of individuals worldwide.

Known monitoring methods and products based on PPG-dedicated algorithms are configured for distinguishing sinus (normal) rhythm episodes from AF ones. Typically, sinus and AF episodes are separated from each other by estimating electrocardiogram (ECG)-based and PPG-based RR intervals. The standard deviation of RR intervals is low during sinus rhythm or high during AF. The inverse is observed with average values of RR intervals which are high during sinus rhythm or low AF. For instance, A.G. Bonomi, et. al., "Atrial Fibrillation Detection Using a Novel Cardiac Ambulatory Monitor Based on Photo-Plethysmography at the Wrist", Journal of the American Heart Association, Aug 7; 7(15) (2018), the describes AF detection using photo-plethysmography signals measured from a wrist-based wearable device.

Document US2018279891A1 discloses a method for event detection in a user-wearable device including receiving, from a first sensor implemented in the user-wearable device, PPG signals; processing, at a processor, the PPG signals to obtain PPG signal samples; detecting, at the processor, beats in the PPG signal samples; dividing the PPG signal samples into PPG signal segments; extracting at least one inter-beat interval feature in each PPG signal segment; classifying, at the processor, each PPG signal segment using the extracted IBI feature associated with the PPG signal segment and using a machine learning model; in response to the classifying, generating, at the processor, an event prediction result for the PPG signal segment based on the extracted IBI feature.

Document "IEEE Transactions on Biomedical Circuits and Systems, vol. 9, no. 5 (2015-10-01), pages 662-669", discloses a method for detection of premature ventricular contractions in PPG. The method relies on 6 features, characterising PPG pulse power, and peak-to-peak intervals. A sliding window approach is applied to extract the features, which are later normalized with respect to an estimated heart rate. Artificial neural network with either linear and non-linear outputs is investigated as a feature classifier.

There is a need in a method that can further separate AF episodes from other cardiac arrhythmias, such as premature ventricular and atrial contractions, flutters, supraventricular and ventricular tachycardias, as well as cardiac dysfunctions such as left branch block and AV node reentry.

US2018279891 discloses a method for event detection in a user-wearable device includes receiving, from a first sensor implemented in the user-wearable device, PPG signals; processing, at a processor, the PPG signals to obtain PPG signal samples; detecting, at the processor, beats in the PPG signal samples; dividing the PPG signal samples into PPG signal segments; extracting at least one inter-beat interval (IBI) feature in each PPG signal segment; classifying, at the processor, each PPG signal segment using the extracted IBI feature associated with the PPG signal segment and using a machine learning model; in response to the classifying, generating, at the processor, an event prediction result for the PPG signal segment based on the extracted IBI feature; and displaying the event prediction result at the user-wearable device. In another embodiment, the method further includes extracting morphology based features.

Reference Andrius Sološenko, et. al., "Photoplethysmography-Based Method for Automatic Detection of Premature Ventricular Contractions", IEEE Transactions on Biomedical Circuits and Systems · October 2015, discloses a method for detection of premature ventricular contractions (PVCs) in PPG. The method relies on 6 features, characterizing PPG pulse power, and peak-to-peak intervals. A sliding window approach is applied to extract the features, which are later normalized with respect to an estimated heart rate. Artificial neural network with either linear and non-linear outputs was investigated as a feature classifier.

### Summary

During recent AF-related studies on PPG signals performed by the present inventors, the analysis of the recorded data has pointed at the fact that the addition of features based on variation of PPG pulse morphologies might be necessary to achieve an improved performance to distinguish various types of cardiac arrhythmia. For example, it was observed that, for large number of AF epochs, pathological PPG pulse morphologies were recorded. It was hypothesized that AF leads to changes in hemodynamics such as reduced stroke volume (followed by a reduction in systemic blood pressure) and a pooling of venous blood, resulting in such pathological PPG pulse morphology. Moreover, variations of PPG pulse amplitudes and PPG baseline variations were observed on numerous episodes during sinus rhythm as well as during AF. The variations can reflect stroke volume variations caused by short and long recovery episodes (consecutive short and long RR intervals) combined with the compensation of the sympathetic vasoconstriction due to peripheral pressure increase. This phenomenon is expected to modulate PPG signals and create the observed low frequency component. Although false negative and false positive PPG pulse detections (pulse upstrokes) were observed during AF episodes, the PPG-based RR intervals were similar to ECG-based RR-intervals. Recent studies by the present inventors have shown a high accuracy (e.g. a mean absolute error < 10 ms) when comparing PPG-based vs ECG-based RR intervals.

Following the above AF-related studies, the inventors have concluded that a method that can further separate AF episodes from other cardiac arrhythmias should be based on quantifying the morphology of the PPG pulses and on the classification of the PPG pulse in accordance with their morphologies.

The present disclosure concerns apparatus (20) comprising a pulsatility signal device configured to measure a PPG signal during a measurement time period and a processing device configured to identify individual PPG pulses in the PPG signal during the measurement time period. The processing device is further configured to determine, within the pulse cycle, for each PPG pulse and using a pulse-wave analysis technique, at least a time-related feature comprising a time duration within a pulse, at least a normalized amplitude-related feature comprising an amplitude value of the PPG signal and normalized by another amplitude-related feature, and at least a signal-to-noise ratio (SNR)-related feature related to the SNR characteristics of the PPG pulse. The processing device is further configured to, for each PPG pulse, use a machine learning model in combination with said at least a time-related feature, said at least a normalized amplitude-related feature and said at least a SNR-related feature, to classify each PPG pulse in the PPG signal as "normal", "pathological" or "non-physiological" and output a time series of pulse classes comprising the pulse classes "normal", "pathological" or "non-physiological" each pulse class being associated to a PPG pulse.

The apparatus disclosed herein allows for accurate classification of a measured PPG pulse and cardiac arrhythmia classification. The method disclosed herein allows for detecting atrial fibrillation (AF), classifying arrhythmias and monitoring blood pressure, SpO₂ and sleep and other HRV-derived parameters.

### Brief Description of the Drawings

The invention will be better understood with the aid of the description of an embodiment given by way of example and illustrated by the figures, in which:
Fig. 1a shows a PPG signal measured by a PPG sensor during a time period;
Fig. 1b shows the PPG signal after removing low frequency components;
Fig. 2a shows a step of identifying cardiac contractions (R-wave peaks), cardiac contraction onsets (P-wave onset) and the corresponding cardiac cycle in an ECG signal;
Fig. 2b shows a step of identifying PPG pulse upstrokes, pulse feet and the corresponding pulse cycle in the PPG signal;
Fig. 3 shows a PPG pulse from which time-related features, normalized amplitude-related features and SNR-related features are extracted;
Fig. 4 illustrates schematically a classifying machine learning model inputted with the time-related, normalized amplitude-related and SNR-related features and outputting a classified time series PPG waveform;
Figs. 5a to 5c represents an ECG signal (Fig. 5a) synchronously with the measured PPG signal (Fig. 5b) and corresponding ECG-based and PPG-based RR intervals (Fig. 5c);
Figs. 6a and 6b shows a measured ECG signal with expert classified labels (Fig. 6a) and a PPG signal measured synchronously with the ECG signal (Fig. 6b);
Fig. 7 shows such classified PPG pulses; and
Fig. 8 illustrates an apparatus comprising a pulsatility signal device and a processing device, according to an embodiment.

### Detailed Description of possible embodiments

In describing and claiming the present disclosure, the following terminology will be used.

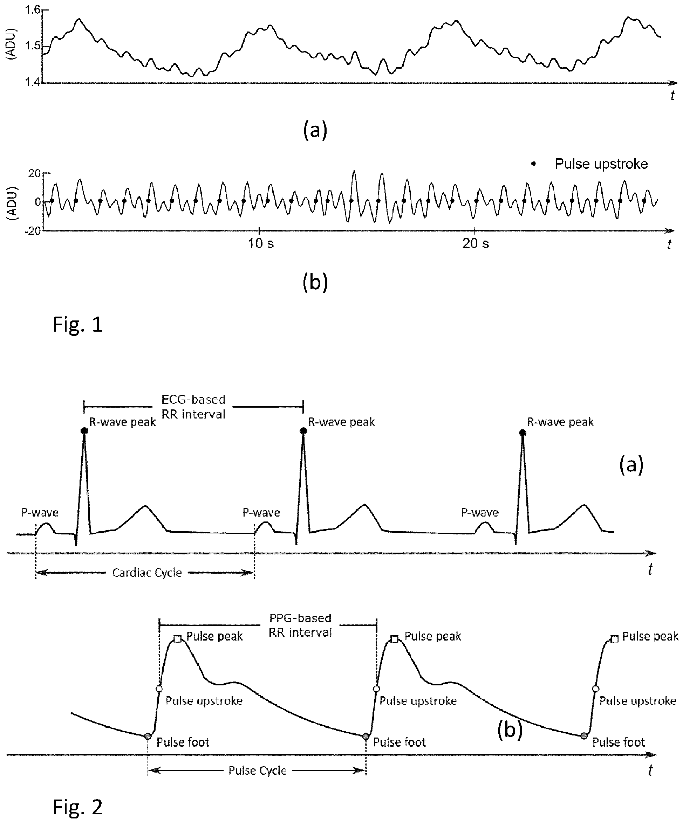

The expression "cardiac contraction" corresponds to the onsets of ventricular contraction of the heart, represented by R-wave peaks in the ECG waveform (black dots in Fig. 2a).

The expression "cardiac cycle" corresponds to the duration between the two successive cardiac contraction onsets (P-wave onsets) of the ECG signal (see Fig. 2a).

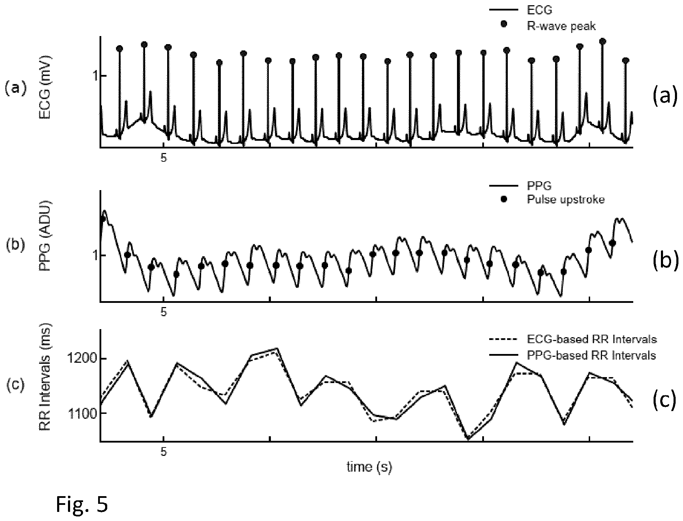

The expression "ECG-based RR intervals" corresponds to the resulting time series representing the time difference between successive cardiac contractions (dashed line in Fig. 5c).

The expression "ECG signal" corresponds to the time series of ECG samples.

The expression "PPG-based RR intervals" corresponds to the resulting time series representing the time difference between successive cardiac contractions (solid line in Fig. 5c). "PPG-based RR intervals" are sometimes also called "PPG-based inter-beat-intervals".

The expression "pulse upstroke" corresponds to the systolic upstrokes in the PPG signals, represented by steep upstrokes (see white dots in Fig. 2b or black dots in Fig. 5b).

The expression "pulse cycle" corresponds to the duration between two successive pulse feet in the PPG signal (see Fig. 2b).

The expression "PPG pulse" corresponds to the PPG signal during a PPG pulse cycle.

The expression "pulse feet" corresponds to the local minima (see gray dots in Fig. 2b) in the PPG signal preceding the pulse upstrokes (see white dots in Fig. 2b) and determine the start of a pulse cycle.

The expression "PPG signal" corresponds to the time series of PPG samples.

The expression "pulse class" corresponds to the resulting classification "normal" (N), "pathological" (P) or "non-physiological" (X) of the PPG pulse provided by a pulse classifier.

The expression "pulse classifier" corresponds to the trained classifying machine learning model.

The expression "time series of pulse classes" corresponds to the output of the pulse classifier which is a time series of PPG classes (N, P or X) each element of the series being associated with a PPG pulse and comprising the temporal occurrence of each pulse.

According to an embodiment, a method based on pulse wave analysis for monitoring cardiovascular vital signs, comprises the steps of:
measuring a PPG signal during a measurement time period such as to obtain a time series of PPG pulses;
during said measurement time period, identifying individual PPG pulses in the PPG signal, wherein each PPG pulse corresponds to the PPG signal during a pulse cycle;
for each PPG pulse, using a pulse-wave analysis technique to determine, within the pulse cycle, at least one of: a time-related feature comprising a time duration, and/or a normalized amplitude-related pulse-related feature, and/or a SNR-related feature; and
for each PPG pulse, using a classifying machine learning model in combination with the determined time-related, normalized amplitude-related and SNR-related features, to classify each PPG pulse in the PPG signal as "normal" (N), "pathological" (P) or "non-physiological" (X) such as to output a time series of pulse classes.

The present disclosure further concerns a computer program comprising instructions for implementing the method for classifying photoplethysmography (PPG) pulses.

In a desired embodiment, the computer program comprises instructions for implementing a method based on pulse wave analysis for monitoring cardiovascular vital signs. The method comprises the steps of:
measuring a PPG signal during a measurement time period such as to obtain a time series of PPG pulses;
during said measurement time period, identifying individual PPG pulses in the PPG signal, wherein each PPG pulse corresponds to the PPG signal during a pulse cycle;
for each PPG pulse, using a pulse-wave analysis technique to determine, within the pulse cycle, at least a time-related feature comprising a time duration within a pulse, at least a normalized amplitude-related feature comprising an amplitude value of the PPG signal and normalized by another amplitude-related feature, and at least a SNR-related feature related to the SNR characteristics of the PPG pulse; and
for each PPG pulse, using a classifying machine learning model in combination with said at least time-related feature, said at least normalized amplitude-related and said at least SNR-related feature, to classify each PPG pulse in the PPG signal as "normal" (N), "pathological" (P) or "non-physiological" (X) such as to output a time series of pulse classes.

The time series of pulse classes comprises the pulse classes "normal" (N), "pathological" (P) or "non-physiological" (X) attributed to each PPG pulse.

Here, the classification "normal" can include a PPG pulse that has been generated by a normal cardiac contraction (cardiac contraction resulting from sinus node depolarization). The classification "pathological" can include a PPG pulse that has been generated by an abnormal cardiac contraction (cardiac contraction not resulting from sinus node depolarization). The classification "non-physiological" can include a PPG pulse associated to a noisy or unidentified PPG pulse. The classification "non-physiological" can further include PPG pulses having a waveform that is not of purely physiological origin (including PPG pulses with motion artefact, electromagnetic perturbation, low SNR, ambient light perturbation). The classification "non-physiological" can further include PPG pulses with physiological sources that are not directly related to arterial pulsatility (e.g. venous pulsatility, ballistocardiographic effect).

**Fig. 1a** shows a PPG signal comprising PPG pulses and measured by PPG sensor (not shown) located in the vicinity of a vascularized tissue, during a time period. The arrival of a blood pressure pulse in small arteries located in the vicinity of the PPG sensor produces an increase of blood volume that results in an augmentation of the light absorption. Here, the PPG signal is considered to have the same orientation as a blood pressure waveform. In this case the measured PPG signal increases when there is an increase in light absorption.

The PPG sensor typically comprises a light source configured to emit a light signal destined to illuminate a vascularized tissue, and a light detector configured to detect the light signal that has illuminated the tissue. The PPG sensor can comprise transmission-based or reflective-based sensor. The light source may comprise any one of a single or multiple light emitting diodes (LED), single or multiple laser diodes (LD), micro-plasma emitters, thermal sources, organic LEDs or tunable lasers. The light detector can comprise any one of photodiodes, phototransistors or any other light sensitive element or a digital camera.

**Fig. 1b** shows a pre-processed PPG signal, for example after removing low frequency components of the PPG signal (also known as baseline wander). For example, the low frequency components can be caused by respiration-induced modulation in stroke volume which is reflected in the PPG signal. In a non-limitative example, the low frequency components can be removed by using a FIR high pass filter with a cut-off frequency at 0.5 Hz. The low frequency components can be removed by removing a baseline wander of the PPG signal. Also shown in Fig. 1b as dots are pulse upstrokes detected from the pre-processed PPG signal. Here, a pulse upstroke corresponds to the timing of a systolic upstroke in the PPG signal.

In one aspect shown in Fig. 2b, the step of identifying pulse upstrokes (white circular dots in Fig. 2b) can be performed by detecting the timing of systolic upstrokes in the PPG signal, in other words by using steepest upwards slope of the PPG signal, for example by using zero-crossings in the second derivative of the PPG signal. Identifying pulse feet can further be performed by detecting local minima preceding the pulse upstrokes in the PPG signal or by any other suitable method. Other fiducial points such as pulse peaks (white squares in Fig. 2b) can be calculated from the PPG signal.

The PPG-based RR intervals can be calculated as the difference between timings of consecutive fiducial points such as pulse upstrokes or pulse feet. Individual PPG pulses (see Fig. 2b) corresponding to the PPG signal during a pulse cycle can then be identified in the PPG signal as the segment of the PPG signal between two successive pulse feet (see gray dots in Fig. 2b), i.e. the local minima preceding the pulse upstrokes (see white dots in Fig. 2b).

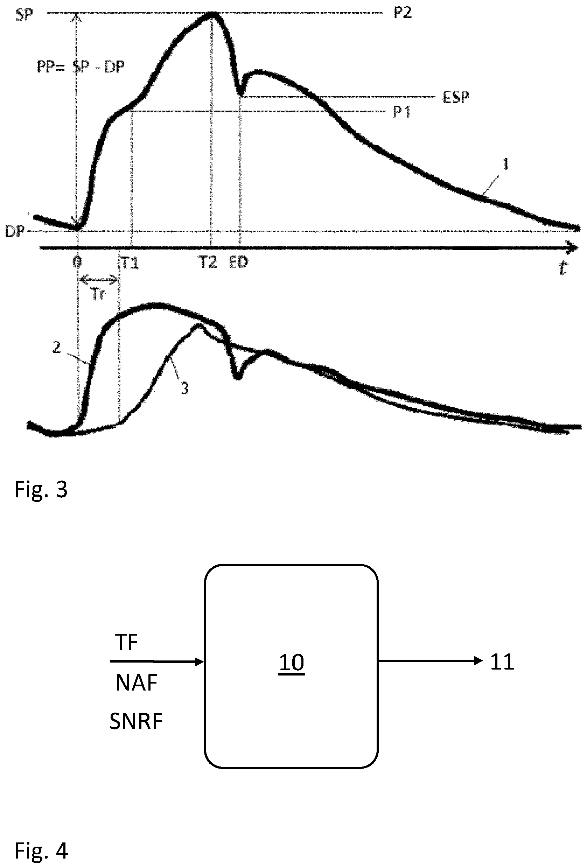

At least one time-related feature (TF) is computed from each PPG pulse of the PPG signal, using a pulse-wave analysis technique **(see** **Fig. 3).** Fig. 3 shows a PPG pulse (pulsatility signal) 1 from which time-related features, normalized amplitude-related features and SNR-related features are extracted. The PPG pulse 1 is the superposition of the forward pulsatility signal 2 generated by a forward pressure wave propagating within an artery, and the backward pulsatility signal 3 generated by a pressure wave reflecting within the artery. The time-related feature can include any time duration within a PPG pulse, the inverse of such a time duration, or any other value computed from such a time duration, e.g. the average of a time duration or its inverse of a plurality of peaks. Examples for such a time-related feature are the time to first peak T1 (duration between the start time of the pulse and its first peak or shoulder of pulse), time to second peak T2 (duration between start time of pulse and second peak or shoulder of pulse), inverse time to first peak 1/T1, inverse of time to second peak 1/T2, time between first and second peak T2-T1, time to reflection Tr (duration between start time of pulse and arrival time of the reflected (backward wave), ejection duration ED (duration between start time of pulse and time of closure of the aortic valve ) or heart rate. Other time-related features can also be contemplated.

At least one normalized amplitude-related feature (NAF) is computed from each PPG pulse of the PPG signal, using a pulse-wave analysis technique (Fig. 3). The normalized amplitude-related feature can be any value based on an amplitude value of the PPG signal and normalized by another amplitude-related feature. Normalization can be performed by a ratio of two amplitude-related features.

The normalized amplitude-related feature can include normalized end-systolic pressure nESP=(ESP-DP)/PP calculated by the difference of the absolute end-systolic pressure ESP and the diastolic pressure DP divided or normalized by the pulse pressure PP. The normalized amplitude-related feature can further include a first augmentation index Alx=(P2-P1)/PP calculated by the difference of the pressure amplitude of the second peak P2 and the pressure amplitude of the first peak P1 divided or normalized by the pulse pressure PP. In one aspect, the normalized amplitude-related feature includes a second augmentation index Alp=(P2-DP)/(P1-DP) calculated by the difference of the pressure amplitude of the second peak P2 and the diastolic pressure amplitude DP divided or normalized by the difference of the pressure amplitude of the first peak P1 and the diastolic pressure amplitude DP. In another aspect, the normalized amplitude-related feature includes a normalized ejection area. The normalized ejection area can be calculated as the surface under the PPG pulse for the ejection duration ED, normalized by the area under the PPG pulse for the duration of this pulse. Other normalized amplitude-related features can also be considered.

At least one SNR-related feature (SNRF) is computed for each PPG pulse of the PPG signal. The SNR is computed for example by computing the number of zero-crossings of the first-time derivative of the PPG pulse. The SNR can be computed using any other suitable method.

**Fig. 4** illustrates schematically the classifying machine learning model 10 inputted with the time-related TF, normalized amplitude NAF and signal-to-noise ratio SNRF related features, and determined for each PPG pulse. The classifying machine learning model 10 classifies each PPG pulse in the pre-processed PPG signal as "normal", "pathological" or "non-physiological" and outputs a time series of PPG pulse classes 11. In other words, the classifying machine learning model 10 automatically assigns a class "normal", "pathological" or "non-physiological" to each PPG pulse of a given unlabeled PPG signal.

In one aspect, time-related TF, normalized amplitude-related NAF and SNR-related features SNRF are determined for the PPG pulse being classified. The TF, NAF, SNRF are determined for the PPG pulse and are then inputted in the classifying machine learning model.

In an embodiment, the method comprises a step of training the classifying machine learning model by using expert-labelled data. The expert-labelled data are typically manually assigned labels by an expert based on an ECG signal. The expert-labelled data should not be confounded by the classes assigned to PPG pulses by classifying machine learning model.

As an example of a supervised learning model, a support vector machine (SVM) can be used to perform the separation between "normal", "pathological", and "non-physiological" pulses based on time-related feature TF, normalized amplitude feature NAF and SNR-related feature SNRF. The SVM defined in Gunn (S. R. Gunn, "Support vector machines for classification and regression," tech. rep., University of Southampton, 1998) can be implemented using a linear kernel function. As mentioned before, the training set is composed of expert labelled pulses of the three classes (N, P, and X). The best training result between penalty coefficient values fixed at one and infinity can be used. The resulting hyperplanes can be applied to PPG-derived features of a single PPG pulse to obtain the mentioned pulse classifier with N, P, or X classes as an independent output for each pulse (see Fig. 4).

A minimum set of specifications must be reached to guaranty a good pulse classifier, namely a minimum set of features and a minimum training set of pulses. For example, for the minimum set of features, the amplitude-related feature nESP (normalized end-systolic pressure) combined with the time-related feature TF (time to first peak T1) and the SNR-related feature SNRF (number of zero-crossings of the first-time derivative of the PPG pulse) can provide good results (see Fig. 3). Concerning the training set of such example, a minimum of 100 samples per subject per class and a minimum of 200 subjects (including patients suffering from different cardiac arrhythmias) can be considered, creating a database of 20'000 individual PPG pulses. The pulse classifier training must be done on the entire database. The resulting pulse classifier is subject-independent, i.e., it can be applied to any subject.

As illustrated in Figs. 5a to 5c, the step of training can comprise measuring an ECG signal (Fig. 5a) synchronously with the PPG signal (Fig. 5b). The cardiac contractions can be identified from the ECG signal (see black dots in Fig. 5a). The pulse upstrokes can be identified from the PPG signal (see black dots in Fig. 5b). Fig. 5c shows ECG-based RR intervals calculated from the ECG signal (dashed line in Fig. 5c) and PPG-based RR intervals calculated from the PPG signal (solid line in Fig. 5c).

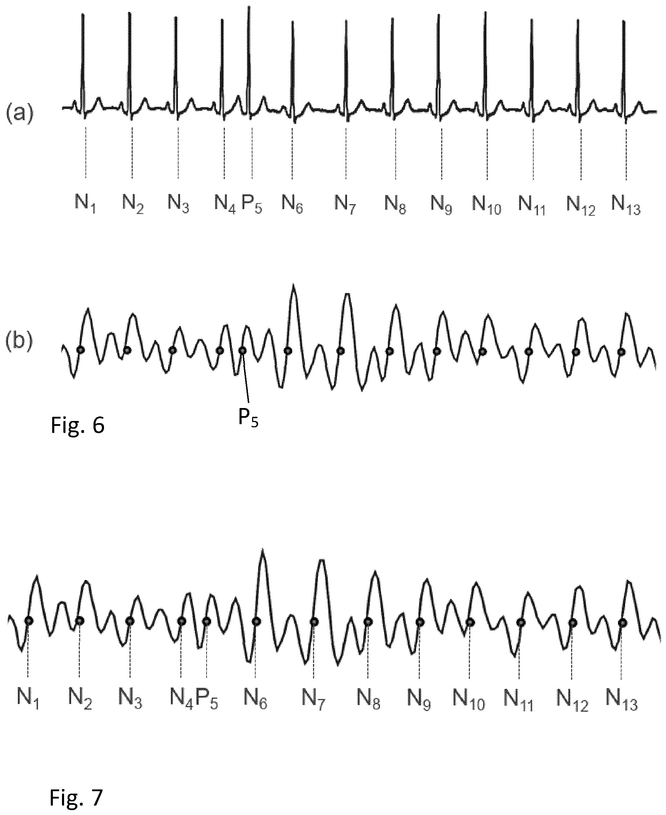

**Fig. 6a** shows an example of an ECG signal for which an expert (or a clinical device) has identified and classified each i^{th} cardiac cycle on the ECG signal as normal (Nᵢ) or pathological (Pᵢ). The ECG signal can be measured by using any ECG setup, e.g., a standard clinical 12-lead setup. The timing of the cardiac contractions is represented by the dots. In Fig. 6a, the expert has classified the fifth cardiac cycle as "pathological" (P₅). The other cardiac cycles are classified as "normal" (N₁ - N₄ and N₆ - N₁₃). The ECG signal of Fig. 6a does not comprise a "non-physiological" cardiac cycle.

**Fig. 6b** shows a PPG signal measured synchronously with the ECG signal. The timing of the pulse upstrokes, obtained by detecting the timing of systolic upstrokes in the PPG signal, are reported on the PPG signals with black dots. The expert classification from the ECG can be used to assign a label for each PPG pulse. Here, the fifth PPG pulse is labelled as "pathological" (P₅) while the other PPG pulses are labelled as "normal".

More generally, expert classification from the ECG signal can be used to assign a "non-physiological" label to PPG pulses which are not associated to a cardiac contraction. For instance, this situation can occur if a PPG pulse is mistakenly detected due to motion artefacts in the PPG signal. During a given period the number of detected pulse cycles can be higher than the number of detected cardiac cycles due to wrongly detected PPG pulses.

Moreover, a "non-physiological" label can be assigned to a PPG pulse that has low SNR characteristics. The "low SNR characteristics" is determined using the SNR-related features during the training of the classifying machine learning model (pulse classifier) using datasets including synchronous PPG and ECG signals (see below). Note that the SNR of a PPG pulse can be determined without requiring measuring an ECG signal.

The remaining non-labeled PPG pulses are either labelled as "normal" or "pathological" or are ignored.

By analyzing the shape of the ECG signal, an expert can identify normal cardiac contractions (originating from the sinoatrial node). Thus, the expert can assign the label "normal" to the PPG pulses that correspond to the ECG pulses analyzed as normal cardiac contractions.

By analyzing the shape of the ECG signal, an expert can identify abnormal, or pathological, cardiac contractions (not originating from the sinoatrial node). The expert can assign the label "pathological" to the PPG pulses that correspond to the ECG pulses analyzed as pathological.

In the case where the ECG signal comprises multiple cardiac contractions but only one single PPG pulse, no label is assigned to the PPG pulse (i.e. due to a missed detection of pulse upstrokes in the PPG or an extra detection of cardiac contractions in the ECG due to the presence of motion or other artifacts in the ECG signal). The PPG pulse is ignored in the training of the classifying machine learning algorithm.

Alternatively or in combination, the expert-labelled data can be obtained from an clinical device (for example a software) which automatically labels cardiac arrhythmia from ECG signals. In that case, the expression "expert" above can be read as "clinical device".

Table 1 shows the different labels applied to the PPG pulses based on the ECG signal expert analysis.

**Table 1**

| **Label** | **ECG** | **PPG** |
|---|---|---|
| Non-physiological (X) | Absence of cardiac cycle | Presence of one PPG pulse with high or low SNR characteristics |
| Non-physiological (X) | Presence of one cardiac cycle with normal or pathological cardiac contraction | Presence of one PPG pulse with low SNR characteristics |
| Normal (N) | Presence of one cardiac cycle with normal cardiac contraction (originating from the sinoatrial node) | Presence of one PPG pulse with high SNR characteristics |
| Pathological (P) | Presence of one cardiac cycle with pathological cardiac contraction (not originating from the sinoatrial node) | Presence of one PPG pulse with high SNR characteristics |
| *(TO BE IGNORED)* | Presence of more than one cardiac cycle | Presence of one single PPG pulse |

The step of training the classifying machine learning model (pulse classifier) by using expert-labelled data can comprise building a dataset including synchronous PPG and ECG signals together with a label attributed to each PPG pulse. The dataset may further include the time-related TF, normalized amplitude-related NAF and SNR-related SNRF features determined from each PPG pulse. The labels, possibly in combination with the time-related TF, normalized amplitude-related NAF and SNR-related SNRF features can be used to train the pulse classifier. The training can use supervised learning models such as support vector machines, decision trees, etc.

The resulting pulse classifier can then be used to classify each PPG pulse based on the time-related TF, normalized amplitude-related NAF and SNR-related SNRF features of the PPG pulse being classified. The pulse classifier further makes use of the statistical distribution of class features (TF, NAF, SNRF) of the preceding PPG pulses to provide an enhanced PPG pulse classification (to manage overlapping between N and P feature spaces).

For each pulse inputted in the pulse classifier, the latter outputs a pulse class. If a time series of PPG pulses is inputted in the pulse classifier, the latter outputs a time series of pulse classes, i.e., a series of individual pulse classes together with the temporal occurrence of each pulse. Each PPG pulse is classified individually as "normal", "pathological" or "non-physiological". **Fig. 7** shows such a time series of pulse classes where one PPG pulse is classified as "pathological" (P₅, extrasystole) and the other PPG pulses are classified as "normal" (N₁ - N₄ and N₆ - N₁₃).

Conventional arrythmia detection can have strong limitations in terms of arrhythmia classifications. For example, conventional arrythmia detection based on PPG-based RR-intervals does not necessarily manage to separate AF episodes from sinus (normal) rhythm episodes with the presence of extrasystoles because both episodes will be characterized by large variations of RR interval values.

The time series of pulse classes, outputted from the pulse classifier, can be used to detect various cardiac arrhythmias and to improve the classification of cardiac arrhythmia.

### Enhanced AF detection

In an embodiment, the method comprises classifying AF. To that end, the classifications "normal" and "pathological" in the time series of classified PPG waveform can be represented as "0" for "normal" and "1" for "pathological". By doing so, one can obtain a time series of digitalized pulse classes (time series comprising the "0" and "1").

In conventional AF detection, a feature extraction algorithm processes RR intervals over time windows of a given duration (e.g. 30 seconds) to estimate features such as: 1) mean value of the RR intervals; 2) minimum value of the RR intervals; 3) maximum value of the RR intervals; 4) median value of the RR intervals and 5) interquartile range of the RR intervals.

The time series of digitalized pulse classes further allows for estimating additional features such as: 6) standard deviation of the digitalized pulse classes and 7) mean value of the digitalized pulse class values.

The set of features can be generated from known episodes of AF and sinus rhythm and can be used to train a classifier (e.g. a support vector machine) to separate set of features values related to AF from set of features values related to sinus rhythm. The trained classifier can be applied to any unknown portion of the PPG signal to detect the presence of AF.

In this example, features 6) and 7) allows for avoiding numerous episodes of false positives (e.g. episodes with extrasystoles).

### Enhanced cardiac arrhythmia classification

In another embodiment, the method comprises classifying cardiac arrhythmias by using the PPG pulses classified as "normal" and "pathological", and not the PPG pulses classified as "non-physiological". Cardiac arrhythmias may include AF, premature ventricular and atrial contractions, flutters, supraventricular and ventricular tachycardias as well as cardiac dysfunctions such as left branch block and AV node reentry.

In one aspect, the method uses the following set of features: time-related features and/or normalized amplitude-related features and/or the SNR-related features and/or the classifications "normal" and "pathological" in the time series of pulse classes. The set of features can be generated from known episodes of cardiac arrhythmias and can be used to train a classifier (e.g. a support vector machine) to separate set of features values related to specific cardiac arrhythmias. The trained classifier can be applied to any unknown portion of the PPG signal to classify cardiac rhyth ms.

Classifying cardiac arrhythmias can be performed for each PPG pulse individually (e.g. for single occurrences of extrasystoles) or for a succession of multiple pulses (e.g. for an episode of AF or trigeminy).

For example, AF episodes are characterized by the presence of pulses classified as "pathological" which appear randomly in between PPG pulses classified as "normal". In opposition, bigeminy, trigeminy episodes are characterized by a regular pattern of PPG pulses classified as "normal" and "pathological" (e.g. for bigeminy, the time series of pulse classes is characterized by a repetition of alternating N and P labels like Nₓ, Pₓ₊₁, Nₓ₊₂, Pₓ₊₃, Nₓ₊₄, ...). Classifying AF can thus comprise identifying random occurrence of the PPG pulses classified as "pathological" in the time series of pulse classes. In one aspect, an episode of AF can be distinguished from a trigeminy event by quantifying the random appearance of the pulses classified as "pathological". Such quantifying can be performed by testing if the distribution of P-to-P intervals is normal (t-test) or not. Here, a p-value of 0.05 can be used as a discriminator.

### Enhanced blood pressure (BP) and oxygen saturation (SpO₂) estimations

In another embodiment, the method comprises normalizing each PPG pulses to obtain normalized PPG pulses. The method further comprises averaging normalized PPG pulses classified as "normal" to obtain enhanced averaged normalized PPG pulses. The enhanced averaged normalized PPG pulses can then be used to estimate a blood pressure value or an SpO₂ value.

### Enhanced HRV feature extraction

In an embodiment, the method comprises determining HRV features by using the PPG pulses classified as "normal" and not the PPG pulses classified as "pathological" and "non-physiological".

The method uses the timing of a given periodic fiducial point in the PPG signal (e.g. pulse upstroke or pulse foot) associated to PPG pulses classified as "normal". The detected timings of the fiducial points classified as "non-physiological" and "pathological" are removed and replaced by interpolated detected timings of neighboring fiducial points classified as "normal". The resulting time series are denoted as enhanced PPG-based NN intervals which represent and indirect measure of the ECG-based NN intervals. In contrast to RR intervals, NN intervals exclude outliers such as ectopic beats and thus only include "normal" RR intervals.

From the ECG-based NN intervals, one can apply classic HRV feature formulas to extract time- and frequency-based features such as the SDNN (standard deviation of all NN intervals), SDANN (standard deviation of the averages of NN intervals in all segments of the entire recording), pNN50 (NN50 count divided by the total number of all NN intervals), VLF (power in the very low frequency range ≤ 0.04 Hz of NN intervals), LF (power in low frequency range (0.04 < *f* ≤ 0.15 Hz) of NN intervals) and HF (power in high frequency range (0.15 < *f* ≤ 0.4 Hz) of NN intervals). The same or any other suitable technique can be applied to the enhanced PPG-based NN intervals leading to enhanced time-based features and enhanced frequency-based HRV features.

### Other HRV-based measurements

In another embodiment, the method comprises determining enhanced time-based features and enhanced frequency-based HRV features from the enhanced PPG-based NN intervals. The method further comprises determining any one of stress level, sleep stages, fatigue/recovery, respiration, circadian cycle, sleep apnoea or other sleep disorders by using the determining enhanced time- and enhanced frequency-based HRV featu res.

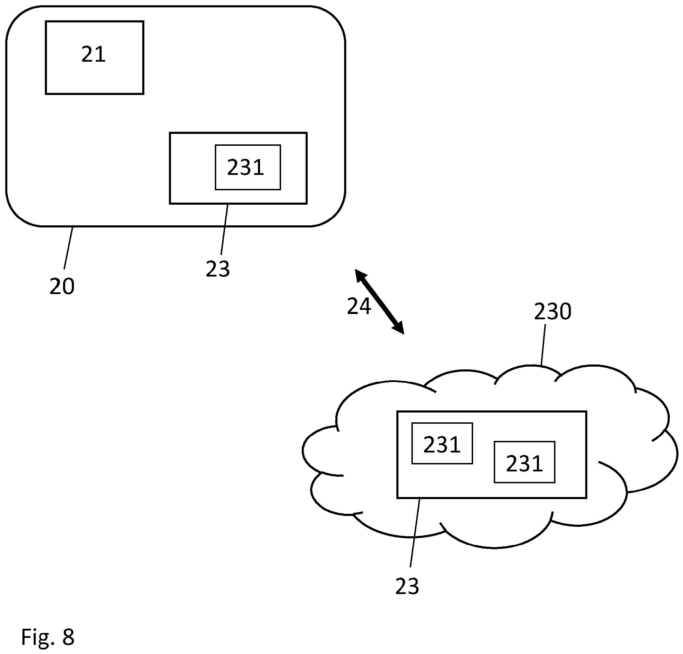

The present disclosure further concerns an apparatus configured to run the computer program. The apparatus is configured to measure a PPG signal during a measurement time period such as to obtain a time series of PPG pulses and for classifying the PPG pulses. **Fig. 8** illustrates an embodiment of the apparatus 20 comprising a pulsatility signal device 21 configured to measure a PPG signal during a measurement time period. The apparatus 20 further comprises a processing device 23 configured to identify individual PPG pulses in the PPG signal during the measurement time period, each PPG pulse corresponding to a pulse cycle. The processing device 23 can be further configured to determine, within the pulse cycle, for each PPG pulse and using a pulse-wave analysis technique, said at least a time-related feature, said at least a normalized amplitude-related feature, and said at least a SNR-related feature. The processing device 23 can be further configured to, for each PPG pulse, using a machine learning model in combination with said at least a time-related feature, said at least a normalized amplitude-related and said at least a SNR-related feature, to classify each PPG pulse in the pre-processed PPG signal as "normal", "pathological" or "non-physiological" and output a time series of pulse classes comprising the pulse classes "normal", "pathological" or "non-physiological" each pulse class being associated to a PPG pulse.

The pulsatility signal device 21 can comprise pulsatility sensor for measuring the pulsatility signal of a user. The pulsatility signal device 21 can be adapted to be in contact with the user's body. For example, the pulsatility signal device 21 can comprise a wearable device (smartwatch, fitness tracker, smart t-shirt). Alternatively, the pulsatility signal device 21 can comprise an implantable device to be implanted into user's body, such as an implant including a PPG sensor. Alternatively, the pulsatility signal device 21 can comprise a remotely sensing device (not shown), such as a camera (including RGB or NIR cameras) performing remote PPG (rPPG) measurements.

The processing device 23 can comprise a single processor 231 or a plurality of processors 231. At least one processor 231 can be comprised (or embedded, for example integrated) in the apparatus 20. Alternatively, or in combination, at least one processor 231 can be remote from the apparatus 20. For example, at least one processor 231 can be comprised in in a remote device 230, such as a cloud computing platform, a smartphone, a personal computer, or any other suitable remote device. In the exampled illustrated in Fig. 8, the processing device 23 includes one processor 231 comprised in the apparatus 20 and two processors 231 comprised in the remote device 230. The remote device 230 can communicate with the apparatus 20 via a wired or wireless communication link 24.

In one aspect, the different calculating steps of identifying individual PPG pulses; determining said at least: a time-related feature, a normalized amplitude-related feature, and a SNR-related feature; and using a machine learning model to classify each PPG pulse can be performed in a distributed manner over the plurality of processors 231.

In another aspect, the steps of identifying individual PPG pulses; determining said at least: a time-related feature, a normalized amplitude-related feature, and a SNR-related feature; can be performed in at least one processor 231 comprised in the apparatus 20, and the step of using a machine learning model to classify each PPG pulse can be performed at least one processor 231 remote from the apparatus 20.

Performing the calculating steps in a distributed manned over the plurality of processors 231 can improve the energy consumption which is of importance for embedded wearable devices.

### Reference numbers

- 1: PPG pulse
- 10: classifying machine learning model
- 11: time series of PPG pulse classes
- 2: forward pulsatility signal
- 20: apparatus
- 21: pulsatility signal device
- 23: processing device
- 230: remote device
- 231: processor
- 24: communication link
- 3: backward pulsatility signal
- 4: PPG sensor

## Claims

1. An apparatus (20) comprising:
a pulsatility signal device (21) configured to measure a PPG signal during a measurement time period,
a processing device (23) configured to identify individual PPG pulses in the PPG signal during the measurement time period;
the processing device (23) being further configured to determine, within the pulse cycle, for each PPG pulse and using a pulse-wave analysis technique, at least a time-related feature comprising a time duration within a pulse, at least a normalized amplitude-related feature comprising an amplitude value of the PPG signal and normalized by another amplitude-related feature, and at least a signal-to-noise ratio (SNR)-related feature related to the SNR characteristics of the PPG pulse;
the processing device (23) is further configured to, for each PPG pulse, use a machine learning model in combination with said at least a time-related feature, said at least a normalized amplitude-related feature and said at least a SNR-related feature, to classify each PPG pulse in the PPG signal as "normal", "pathological" or "non-physiological" and output a time series of pulse classes comprising the pulse classes "normal", "pathological" or "non-physiological" each pulse class being associated to a PPG pulse.

2. The apparatus according to claim 1,
wherein using a machine learning model comprises a step of training the machine learning model by using expert-labelled data; and
wherein the expert-labelled data are assigned by an expert based on an ECG signal and/or obtained from a clinical device which automatically labels cardiac arrhythmia from an ECG signal.

3. The apparatus according to claim 2,
wherein the step of training comprises measuring an ECG signal synchronously with the PPG signal and identifying cardiac contractions from the measured ECG signal;
wherein expert-labelled data comprise labelling PPG pulses which are not associated to a cardiac contraction as "non-physiological"; and
wherein expert-labelled data further comprise labeling PPG pulses which are associated to normal cardiac contraction as "normal", and
labeling PPG pulses which are associated to pathological cardiac contraction as "pathological".

4. The apparatus according to claim 2 or 3,
wherein expert-labelled data further comprise labeling PPG pulses which are associated to low SNR characteristics as "non-physiological.

5. The apparatus according to any one of claims 1 to 4,
wherein the PPG pulses classified as "normal" and "pathological", and not the PPG pulses classified as "non-physiological" are used to classify cardiac arrhythmias.

6. The apparatus according to claim 5,
wherein classifying cardiac arrhythmias include atrial fibrillation (AF), extrasystoles and tachycardia, premature ventricular and atrial contractions, flutters, supraventricular and ventricular tachycardias as well as cardiac dysfunctions such as left branch block and AV node reentry.

7. The apparatus according to any one of claims 1 to 4,
wherein identifying random occurrence of the "pathological" pulse class in the time series of pulse classes are used to detect AF.

8. The apparatus according to any one of claims 1 to 4,
wherein normalized PPG pulses comprises normalizing each PPG pulses;
wherein normalized PPG pulses classified as "normal" are averaged to obtain enhanced averaged normalized PPG pulses; and
wherein the enhanced averaged normalized PPG pulses are used to estimate a blood pressure value or a SpO₂ value.

9. The apparatus according to any one of claims 1 to 4,
wherein the time series of "normal" pulses classes and not the PPG pulses classified as "pathological" and "non-physiological" are used to determine heart rate variability (HRV) features.

10. The apparatus according to claim 9,
wherein a timing of each PPG pulse classified as "normal" is identified in the time series of pulse classes, wherein timings of "non-physiological" and "pathological" pulse classes are replaced by interpolated timings of nearest "normal" PPG pulses resulting in enhanced PPG-based NN intervals; and
enhanced time-based features and enhanced frequency-based HRV features are determined from the timing of the enhanced PPG-based NN intervals.

11. The apparatus according to any one of claims 1 to 10,
wherein said identifying individual PPG pulses comprises detecting fiducial points in the PPG signal.

12. The apparatus according to any one of claims 1 to 11,
wherein the method comprises digitalizing the time series of pulse classes as "0" for "normal" and "1" for "pathological"; estimating standard deviation and mean value of the digitalized time series of pulse class is estimated; and using the estimated standard deviation and mean value to detect Af.

13. The apparatus according to any one of claims 1 to 12,
wherein said at least a time-related features comprises the time to first peak (T1).

14. The apparatus according to any one of claims 1 to 13,
wherein said at least a normalized amplitude-related features comprises the normalized end-systolic pressure (nESP).

15. The apparatus according to any one of claims 1 to 14,
wherein said at least a SNR-related features comprises the number of zero-crossings of the first-time derivative of the PPG pulse.

16. The apparatus according to any one of claims 1 to 15,
wherein said machine learning model comprises a support vector machine configured to perform the separation between "normal", "pathological", and "non-physiological" pulses based on said at least a time-related feature, said at least a normalized amplitude-related and said at least a SNR-related feature.

17. The apparatus according to any one of claims 1 to 16,
wherein the processing device (23) comprises at least one processor (231) comprised in the apparatus (20) and at least one processor (231) remote from the apparatus (20); and
wherein the steps of identifying individual PPG pulses; determining said at least: a time-related feature, a normalized amplitude-related feature, and a SNR-related feature; are performed in said at least one processor (231) comprised in the apparatus (20); and the step of using a machine learning model to classify each PPG pulse is performed in said at least one processor (231) remote from the apparatus (20).

## Patentansprüche

1. Vorrichtung (20), umfassend:
eine Pulsatilitätssignalvorrichtung (21), welche dazu konfiguriert ist, ein PPG-Signal während eines Messzeitraums zu messen,
eine Verarbeitungsvorrichtung (23), welche dazu konfiguriert ist, einzelne PPG-Impulse im PPG-Signal während des Messzeitraums zu identifizieren;
wobei die Verarbeitungsvorrichtung (23) zudem dazu konfiguriert ist, innerhalb des Pulszyklus für jeden PPG-Impuls und unter Verwendung einer Pulswellenanalysetechnik mindestens ein zeitbezogenes Merkmal zu bestimmen, welches eine Zeitdauer innerhalb eines Pulses umfasst, mindestens ein normalisiertes amplitudenbezogenes Merkmal zu bestimmen, welches einen Amplitudenwert des PPG-Signals umfasst und durch ein anderes amplitudenbezogenes Merkmal normalisiert ist, und mindestens ein Signal-Rausch-Verhältnis (SNR)-bezogenes Merkmal zu bestimmen, welches mit den SNR-Eigenschaften des PPG-Impulses in Zusammenhang steht;
wobei die Verarbeitungsvorrichtung (23) zudem dazu konfiguriert ist, für jeden PPG-Impuls eine maschinelles Lernmodell zu verwenden in Kombination mit dem besagten mindestens einen zeitbezogenen Merkmal, dem besagten mindestens einen normalisierten amplitudenbezogenen Merkmal und dem besagten mindestens einen SNR-bezogenen Merkmal, um jeden PPG-Impuls als "normal", "pathologisch" oder "nicht-physiologisch" zu klassifizieren und eine Zeitreihe von Impulsklassen auszugeben, welche die Impulsklassen "normal", "pathologisch" oder "nicht-physiologisch" umfassen, wobei jede Impulsklasse einem PPG-Impuls zugeordnet ist.

2. Vorrichtung gemäss Anspruch 1, worin die Verwendung eines maschinellen Lernmodells einen Schritt des Trainierens des maschinellen Lernmodells umfasst, worin von Experten gekennzeichneten Daten verwendet werden; und worin die von Experten gekennzeichneten Daten von einem Experten auf Basis eines EKG-Signals zugewiesen und/oder von einem klinischen Gerät erhalten werden, welches Herzrhythmusstörungen automatisch anhand eines EKG-Signals kennzeichnet.

3. Vorrichtung gemäss Anspruch 2, worin der Schritt des Trainierens das Messen eines EKG-Signals synchron mit dem PPG-Signal und das Identifizieren von Herzkontraktionen aus dem gemessenen EKG-Signal umfasst; worin die von Experten gekennzeichnete Daten das Kennzeichnen von PPG-Impulsen, welche nicht mit einer Herzkontraktion in Zusammenhang stehen, als "nicht physiologisch" umfassen; und worin die von Experten gekennzeichnete Daten zudem das Kennzeichnen von PPG-Impulsen, welche mit einer normalen Herzkontraktion in Zusammenhang stehen, als "normal" und das Kennzeichnen von PPG-Impulsen, welche mit einer pathologischen Herzkontraktion in Zusammenhang stehen, als "pathologisch", umfassen.

4. Vorrichtung gemäss Anspruch 2 oder 3, worin die von Experten gekennzeichneten Daten zudem die Kennzeichnung von PPG-Impulsen, welche mit niedrigen SNR-Eigenschaften verbunden sind, als "nicht physiologisch" umfassen.

5. Vorrichtung gemäss irgendeinem der Ansprüche 1 bis 4, worin die als "normal" und "pathologisch" klassifizierten PPG-Impulse und nicht die als "nicht-physiologisch" klassifizierten PPG-Impulse für das Klassifizieren von Herzrhythmusstörungen verwendet werden.

6. Vorrichtung gemäss Anspruch 5, worin die Klassifizierung von Herzrhythmusstörungen Vorhofflimmern (AF), Extrasystolen und Tachykardie, vorzeitige ventrikuläre und atriale Kontraktionen, Herzflattern, supraventrikuläre und ventrikuläre Tachykardien sowie Herzfunktionsstörungen wie Linksastblock und AV-Knoten-Reentry umfasst.

7. Vorrichtung gemäss irgendeinem der Ansprüche 1 bis 4, worin das Erkennen von zufälligem Auftreten der "pathologischen" Pulsklasse in der Zeitreihe der Pulsklassen für das Erkennen von Vorhofflimmern (AF) verwendet wird.

8. Vorrichtung gemäss irgendeinem der Ansprüche 1 bis 4, worin normalisierte PPG-Impulse das Normalisieren jedes PPG-Impulses umfassen; worin als "normal" klassifizierte normalisierte PPG-Impulse gemittelt werden, um verbesserte gemittelte normalisierte PPG-Impulse zu erhalten; und worin die verbesserte gemittelte normalisierte PPG-Impulse zum Schätzen eines Blutdruckwerts oder eines SpO₂-Werts verwendet werden.

9. Vorrichtung gemäss irgendeinem der Ansprüche 1 bis 4, worin die Zeitreihen von "normalen" Pulsklassen und nicht von als "pathologisch" und "nicht-physiologisch" klassifizierten PPG-Pulsen für das Bestimmen von HRV-Merkmalen verwendet werden.

10. Vorrichtung gemäss Anspruch 9, worin der Zeitpunkt jedes der als "normal" klassifizierten PPG-Impulses in der Zeitreihe der Impulsklassen identifiziert wird, worin Zeitpunkte von "nicht-physiologischen" und "pathologischen" Impulsklassen durch interpolierte Zeitpunkte der nächsten "normalen" PPG-Impulse ersetzt werden, was zu verbesserten PPG-basierten NN-Intervallen führt; und verbesserte zeitbasierte Merkmale und verbesserte frequenzbasierte HRV-Merkmale aus dem Zeitpunkt der verbesserten PPG-basierten NN-Intervalle bestimmt werden.

11. Vorrichtung gemäss irgendeinem der Ansprüche 1 bis 10, worin das besagte Identifizieren einzelner PPG-Impulse das Erkennen von Bezugspunkten im PPG-Signal umfasst.

12. Vorrichtung gemäss irgendeinem der Ansprüche 1 bis 11, worin das Verfahren das Digitalisieren der Zeitreihe von Pulsklassen als "0" für "normal" und "1" für "pathologisch", das Schätzen der Standardabweichung und des Mittelwerts der digitalisierten Zeitreihe von Pulsklassen und das Verwenden der geschätzten Standardabweichung und des Mittelwerts zum Erkennen von AF umfasst.

13. Vorrichtung gemäss irgendeinem der Ansprüche 1 bis 12, worin das besagte mindestens eine zeitbezogene Merkmal die Zeit bis zum ersten Peak (T1) umfasst.

14. Vorrichtung gemäss irgendeinem der Ansprüche 1 bis 13, worin das mindestens eine normalisierte amplitudenbezogene Merkmal den normalisierten endsystolischen Druck (nESP) umfasst.

15. Vorrichtung gemäss irgendeinem der Ansprüche 1 bis 14, worin das besagte mindestens eine SNR-bezogene Merkmal die Anzahl der Nulldurchgänge der ersten Zeitableitung des PPG-Impulses umfasst.

16. Vorrichtung gemäss irgendeinem der Ansprüche 1 bis 15, worin das maschinelle Lernmodell eine Support Vector Machine umfasst, welche dazu konfiguriert ist, die Trennung zwischen "normalen", "pathologischen" und "nicht-physiologischen" Impulsen basierend auf dem besagten mindestens einen zeitbezogenen Merkmal, dem besagten mindestens einen normalisierten amplitudenbezogenen Merkmal und dem besagten mindestens einen SNR-bezogenen Merkmal vorzunehmen.

17. Vorrichtung gemäss irgendeinem der Ansprüche 1 bis 16, worin die Verarbeitungsvorrichtung (23) mindestens einen in der Vorrichtung (20) enthaltenen Prozessor (231) und mindestens einen von der Vorrichtung (20) entfernten Prozessor (231) umfasst; und worin die Schritte des Identifizierens einzelner PPG-Impulse; des Bestimmens von mindestens einem zeitbezogenen Merkmal, einem normalisierten amplitudenbezogenen Merkmal und einem SNR-bezogenen Merkmal im besagten mindestens einen in der Vorrichtung (20) enthaltenen Prozessor (231) ausgeführt werden; und worin der Schritt des Verwendens eines maschinellen Lernmodells zum Klassifizieren jedes PPG-Impulses im besagten mindestens einen von der Vorrichtung (20) entfernten Prozessor (231) ausgeführt wird.

## Revendications

1. Appareil (20) comprenant :
un dispositif de signal de pulsatilité (21) configuré pour mesurer un signal PPG pendant une période de mesure,
un dispositif de traitement (23) configuré pour identifier des impulsions PPG individuelles dans le signal PPG pendant la période de mesure,
le dispositif de traitement (23) étant en outre configuré pour déterminer, dans le cycle d'impulsion, pour chaque impulsion PPG et en utilisant une technique d'analyse d'onde d'impulsion, au moins une caractéristique liée au temps comprenant une durée dans une impulsion, au moins une caractéristique liée à l'amplitude normalisée comprenant une valeur d'amplitude du signal PPG et normalisé par une autre caractéristique liée à l'amplitude, et au moins une caractéristique liée au rapport signal/bruit (SNR) liée aux caractéristiques SNR de l'impulsion PPG
le dispositif de traitement (23) étant en outre configuré pour utiliser, pour chaque impulsion PPG, un modèle d'apprentissage automatique en combinaison avec ladite au moins une caractéristique liée au temps, ladite au moins une caractéristique liée à l'amplitude normalisée et ladite au moins une caractéristique liée au SNR, pour classer chaque impulsion PPG dans le signal PPG comme "normal", "pathologique" ou "non physiologique" et émettre une série temporelle de classes d'impulsions comprenant les classes d'impulsions "normales", "pathologiques" ou "non physiologiques", chaque classe d'impulsions étant associée à une impulsion PPG.

2. Appareil selon la revendication 1, dans lequel l'utilisation d'un modèle d'apprentissage automatique comprend une étape consistant à entraîner le modèle d'apprentissage automatique en utilisant des données étiquetées par des experts ; et dans lequel les données étiquetées par un expert sont attribuées par un expert sur la base d'un signal ECG et/ou obtenues à partir d'un dispositif clinique qui étiquette automatiquement l'arythmie cardiaque à partir d'un signal ECG.

3. Appareil selon la revendication 2, dans lequel l'étape d'entraînement comprend la mesure d'un signal ECG de manière synchrone avec le signal PPG et l'identification des contractions cardiaques à partir du signal ECG mesuré ; dans lequel les données étiquetées par des experts comprennent l'étiquetage des impulsions PPG qui ne sont pas associées à une contraction cardiaque comme étant "non physiologiques"; et dans lequel les données étiquetées par des experts comprennent en outre l'étiquetage des impulsions PPG qui sont associées à une contraction cardiaque normale comme étant "normales", et l'étiquetage des impulsions PPG qui sont associées à une contraction cardiaque pathologique comme étant "pathologiques".

4. Appareil selon la revendication 2 ou 3, dans lequel les données étiquetées par des experts comprennent en outre l'étiquetage des impulsions PPG qui sont associées à de faibles caractéristiques SNR comme étant "non physiologiques".

5. Appareil selon l'une quelconque des revendications 1 à 4, dans lequel les impulsions PPG classées comme "normales" et "pathologiques", et non les impulsions PPG classées comme "non physiologiques" sont utilisées pour classifier les arythmies cardiaques.

6. Appareil selon la revendication 5, dans lequel la classification des arythmies cardiaques comprend la fibrillation auriculaire (FA), les extrasystoles et la tachycardie, les contractions ventriculaires et auriculaires prématurées, les flutters, les tachycardies supraventriculaires et ventriculaires ainsi que les dysfonctionnements cardiaques tels qu'un bloc de branche gauche et une réentrée du noeud AV.

7. Appareil selon l'une quelconque des revendications 1 à 4, dans lequel l'identification de l'apparition aléatoire de la classe d'impulsions "pathologiques" dans la série temporelle de classes d'impulsions est utilisée pour détecter une fibrillation auriculaire (FA).

8. Appareil selon l'une quelconque des revendications 1 à 4, dans lequel les impulsions PPG normalisées comprennent la normalisation de chaque impulsion PPG ; dans lequel la moyenne des impulsions PPG normalisées classées comme "normales" est calculée pour obtenir des impulsions PPG normalisées moyennes améliorées ; et dans lequel les impulsions PPG normalisées moyennes améliorées sont utilisées pour estimer une valeur de pression artérielle ou une valeur SpO₂.

9. Appareil selon l'une quelconque des revendications 1 à 4, dans lequel les séries temporelles de classes d'impulsions "normales" et non les impulsions PPG classées comme "pathologiques" et "non physiologiques" sont utilisées pour déterminer les caractéristiques de variabilité du rythme cardiaque VRC.

10. Appareil selon la revendication 9, dans lequel un timing de chaque impulsion PPG classée comme "normale" est identifié dans la série temporelle de classes d'impulsions, dans lequel les timings des classes d'impulsions "non physiologiques" et "pathologiques" sont remplacés par des temporisations interpolées des des impulsions PPG "normales" les plus proches, ce qui entraîne des intervalles NN améliorés basés sur PPG ; et des caractéristiques améliorées basées sur le temps et des caractéristiques améliorées de VRC basées sur la fréquence sont déterminées à partir du timing des intervalles NN améliorés basés sur PPG.

11. Appareil selon l'une quelconque des revendications 1 à 10, dans lequel ladite identification d'impulsions PPG individuelles comprend la détection de points de repère dans le signal PPG.

12. Appareil selon l'une quelconque des revendications 1 à 11, dans lequel le procédé comprend la numérisation de la série temporelle de classes d'impulsions comme "0" pour " normal" et "1" pour "pathologique" ; l'estimation de l'écart type et la valeur moyenne de la série temporelle numérisée de la classe d'impulsions ; et l'utilisation de l'écart type estimé et la valeur moyenne pour détecter la FA.

13. Appareil selon l'une quelconque des revendications 1 à 12, dans lequel ladite au moins une caractéristique liée au temps comprend le temps jusqu'au premier pic (T1).

14. Appareil selon l'une quelconque des revendications 1 à 13, dans lequel ladite au moins une caractéristique normalisée liée à l'amplitude comprend la pression télésystolique normalisée (nESP).

15. Appareil selon l'une quelconque des revendications 1 à 14, dans lequel ladite au moins une caractéristique liée au SNR comprend le nombre de passages par zéro de la dérivée première de l'impulsion PPG.

16. Appareil selon l'une quelconque des revendications 1 à 15, dans lequel ledit modèle d'apprentissage automatique comprend une machine à vecteurs de support configurée pour effectuer la séparation entre les impulsions "normales", "pathologiques" et "non physiologiques" sur la base de ladite au moins une caractéristique liée au temps, ladite au moins une caractéristique liée à l'amplitude normalisée et ladite au moins une caractéristique liée au SNR.

17. Appareil (20) selon l'une quelconque des revendications 1 à 16, dans lequel le dispositif de traitement (23) comprend au moins un processeur (231) compris dans l'appareil (20) et au moins un processeur (231) éloigné de l'appareil (20) ; et dans lequel les étapes consistant à identifier des impulsions PPG individuelles ; déterminer les au moins une lesdites caractéristiques : une caractéristique liée au temps, une caractéristique liée à l'amplitude normalisée et une caractéristique liée au SNR ; sont effectuées dans ledit au moins un processeur (231) compris dans l'appareil (20) ; et l'étape consistant à utiliser un modèle d'apprentissage automatique pour classer chaque impulsion PPG est effectuée dans ledit au moins un processeur (231) éloigné de l'appareil (20).
